# EUROPEAN PATENT APPLICATION

(11) **EP 2 108 322 A1**
(43) Date of publication of application: **14.10.2009**
(21) Application number: 08075286.8
(22) Date of filing: 09.04.2008
(51) Int. Cl.: A61B 17/74, A61B 17/78, A61B 17/16, A61B 17/76, A61B 17/80, A61B 17/86

(54) **Trochanteric contention/compression plate**

(71) Applicant: Costa Martins, José, 2820-590 Ch. Caparica (PT)
(72) Inventor: Costa Martins, José, 2820-590 Ch. Caparica (PT)

(57) **Abstract**

The present appliance, mentions a device designed for plate Trochanteric of Corrtention/compression (1), that for its form it allows to the treatment of the Intertrochanteric fractures, some types of subtrochanteric fractures and femoral neck fractures with necrosis of the head, having the capacity to serve of jamb in the interior of the bone in these types of fractures, like a nail, keeping the length of the neck of the Femur to the level of the habitually fragmented Intertrochanteric or subtrochanteric zone.

In case of a femoral neck fracture with necrosis of the head, wo can change the necrosis head for a metallic prosthesis of the head (16) to screw on the femoral neck screw (3).

## Description

The present device, mentions a system consisting in a trochantheric plate of dampening/compression (1) that has his characteristics in its form, a part internally to the bone in the trochanteric region and another one external to the shaft. It has a screw of landslide (3) in the axle of the neck of the Femur, that allows the compression to the level of the fracture in the trochanteric region, being the plate pressed to the cortical of the shaft of the femur for screws (4) that are screwed in the cited plate. It is used for treatment of Intertrochanteric and in some types of subtrochanteric fractures, and for its characteristics it serves of internal support, like a nail, in the internal portion of the plate, not allowing the loss of length of the neck of the Femur in the fractured and habitually fragmented region. On conclusion its originality and principle of treatment in the level of the fragmented fracture in the trochanteric region is based on the function of internal support to the bone, also allowing compression at the same level. In case of a femoral neck fracture with necrosis of the head, we can change the necrosis head for a metallic prosthesis of the head (16) to screw on the femoral neck screw (3).

The present device now goes to be explained with detail, with the aid of the attached drawings:
- fig. 1 is the plate of dampening/compression;
- fig. 2 is the screw of compression of the neck of the femur placed in the plate;
- fig. 3 is the screw of compression of the neck of the femur, with one cut in the proximal half portion (a) where is a grooves for brake screw and puncture for K. wire way, it as a distal exterior threaded for the head of the Femur and proximal interior for squeeze of its system of application;

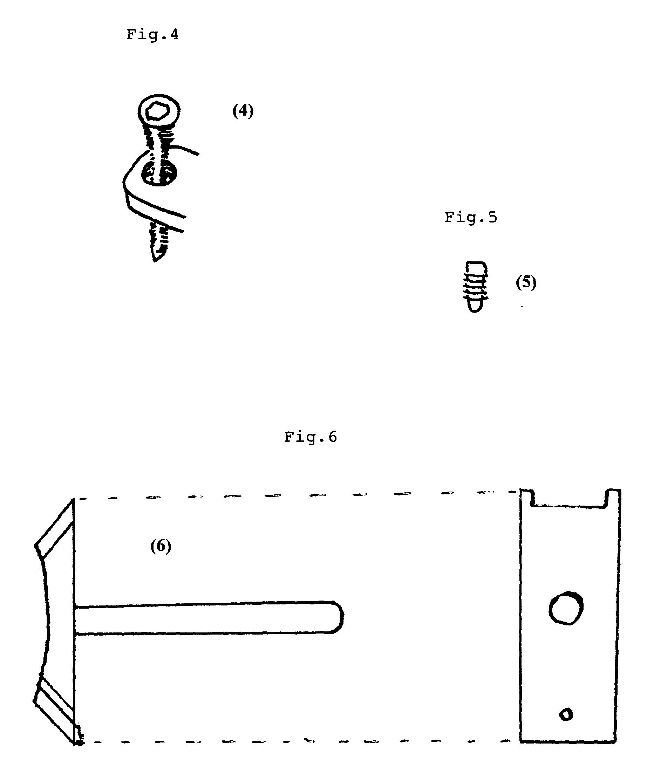

- fig. 4 is the screw of squeeze of the plate to shaft of the femur, has a conic form near the head for squeeze to the plate;
- fig.5 is the screw of brake of the screw of compression of the neck of the femur;
- fig. 6 is the system of initial orientation of the screw of compression of the neck of the femur and the rank of the plate;

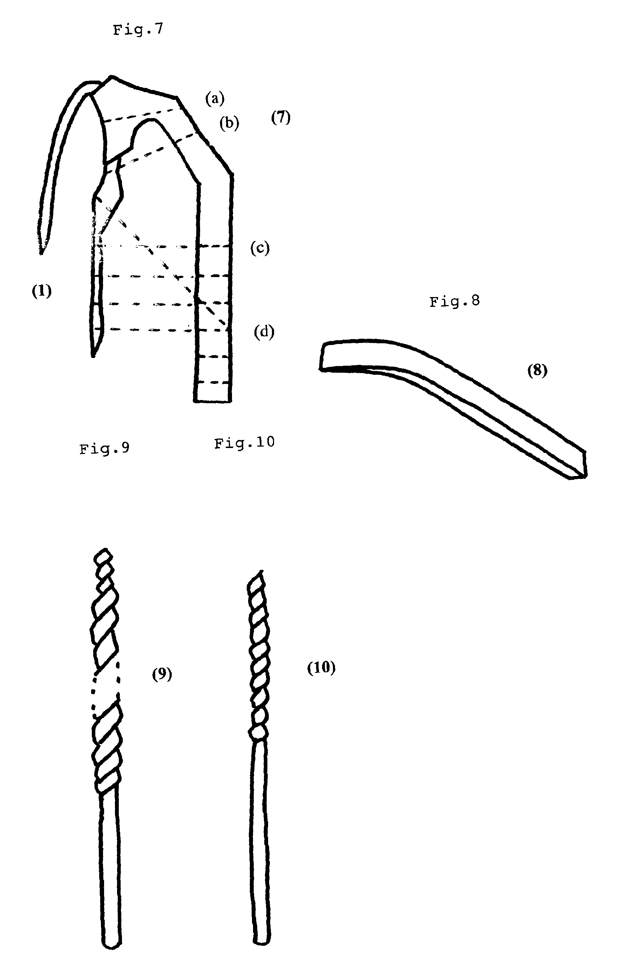

- fig. 7 is the system of application of the plate and screws (the hatched lines are the orientation of the diverse punctures);
- fig. 8 is the osteotome for opening of the entrance of the plate in the intertrochanteric region;
- fig. 9 is the drill cannulated for the screw of the neck of the Femur, has the tip with lesser diameter;
- fig. 10 is the drill for the screws of squeeze of the plate to the shaft;

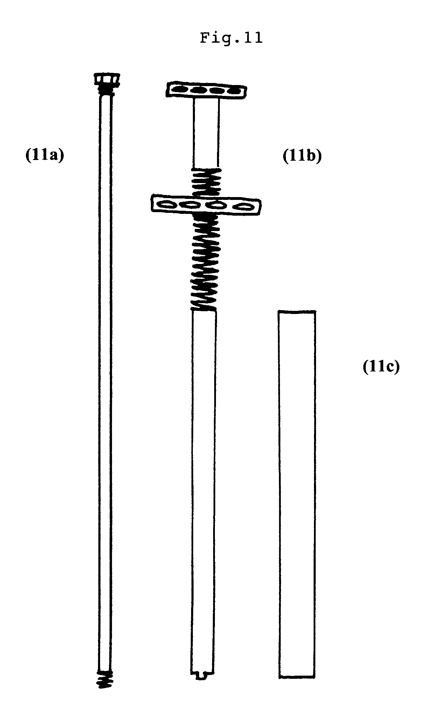

- fig. 11 is the system of application (11 a), compression (11 b) and tube of protection (11 c) for the neck screw;

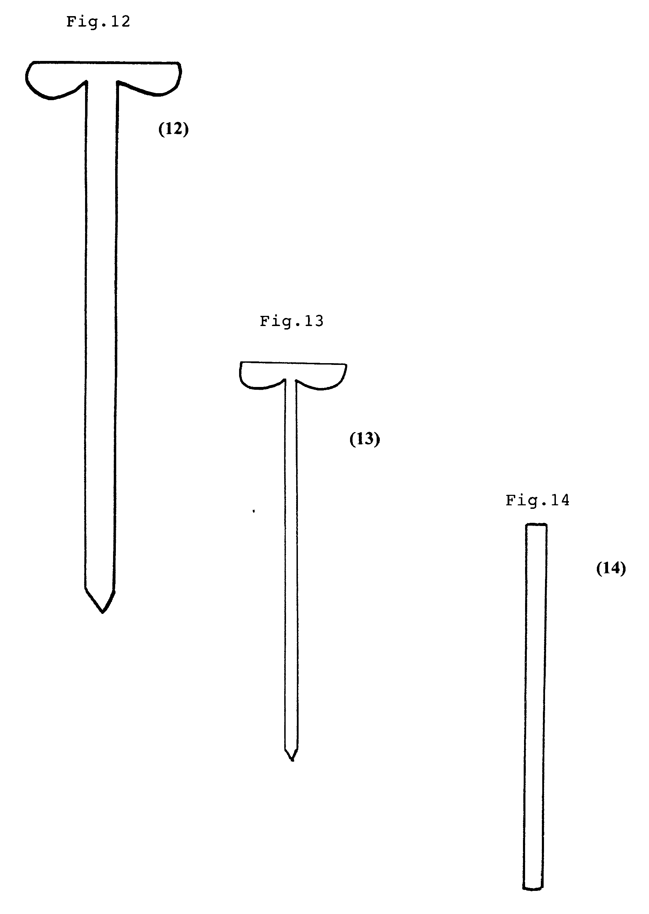

- fig. 12 is the great Trocart;
- fig. 13 is the small Trocart;
- fig. 14 is the tube of protection of the screws of the plate;
- fig. 15 is the plate applied to the Femur
- fig. 16 is the femoral head prosthesis with the plate

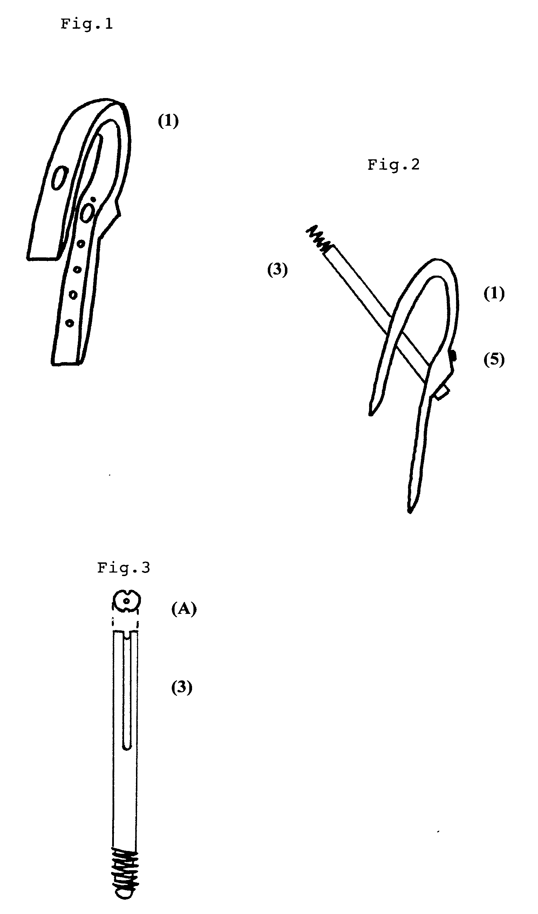

### Description of the assembly of the plate of contention/compression (fig.1):

It is initiated puting a K. wire to the neck of the Femur in his axel, from the trochanteric region, using the system of initial orientation (6). After that and with the same system, is marked the opening for the introduction of the internal portion of the plate, with a drill.

We take off the K. wire.

After that, we use a proper osteotome for orientation of the introduction of the related plate (8). We take off the initial system of orientation (6) and we placed the plate of Dampening/contention (1) with his application system (7). It has the perforations (a) for squeeze of the system of application (7) to the plate (1), (b) for braking screw (5), (c) for screw of squeeze at the plate (1) to shaft of the Femur and (d) for introduction of the screw of compression of the neck of the Femur (3).

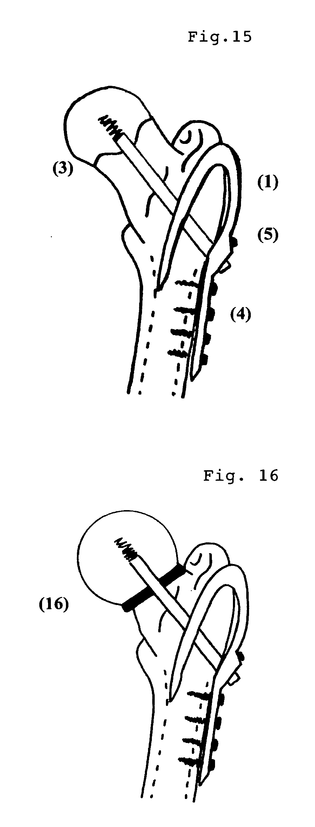

It is placed through the tube of protection of the system (11c) after initial perforation with great Trocart (12), the wire guides (K. wire existing in the market) for the screw of compression of the neck. Through it with a cannulated drill (9) we perforate the neck and placed the related screw (3). After that we take off the K. wire. This rank becomes through the application system (11a), being this pressed to the screw of the neck (3) and placed inside of the compression system (11b) that it functions inside of the tube of protection (11c). Later the brake of tho seeding of the screw of the neck is made, with proper screw (5). With the drill aid (10), after initial perforation with the small Trocart (13) and through its tube of protection (14) is opened entered of the screws of the plate, being these (4) pressed to the plate and shaft of the Femur, being finished the surgery (Fig. 15).

In case of a femoral neck fracture with necrosis of the head, we can change the necrosis head for a metallic prosthesis of the head (16) to screw on the femoral neck screw (3).

## Claims

**1.** Trochanteric plate of contention/compression device, for treatment of Intertrochanteric fractures, some subtrochanteric and femoral neck fractures with necrosis, **characterized** for being composed for a plate in a "U" form, used inverted, being a part of this used internally to the bone and another part used external to the bone (1), allowing its internal part, serving of support, containing the impaction of the fracture in its fragmented part, not allowing the loss of length of the neck of the Femur (15).

**2.** Device plate that in accordance with the claim n° 1, **characterized** for including a screw (3) that it is used through the plate (1) and is placed in the axle of the neck of the Femur to keep the same in the adequate angle to the shaft and that it also allows the necessary compression in the fractured region.

**3.** Device plate that in accordance with the claim n° 1 **characterized** for in case of a femoral neck fracture with necrosis of the head, we can change the necrosis head for a metallic prosthesis of the head (16) to screw on the femoral neck screw (3).

## Amended claims

### Amended claims in accordance with Rule 137(2) EPC.

**1.** Trochanteric plate of contention-compression for treatment of Intertrochanterics fractures and some sub-trochanterics, of the type of the ones that are composed for a plate in form of "U" used in an inverted form, with one of the sides most long, **characterized** for understanding:
- a plate (1) whose shorter side has a puncture and is introduced in the interior of the bone, having the function of the contention and controlling the landslide of the neck of femur and whose more long side has 4 or more punctures of setting to the bone;
- a boost glide screw (3) that it crosses in the diagonal line both the sides of the plate in "U";
- four or more screws that adjust the most long side of the "U" plate to the cortical of femoral diaphysis; and
- a screw (5) of braking of the boost glide screw (3).
